# EUROPEAN PATENT SPECIFICATION

(11) **EP 1 303 276 B1**
(45) Date of publication and mention of the grant of the patent: **06.10.2004**
(21) Application number: 01940893.9
(22) Date of filing: 21.06.2001
(51) Int. Cl.: A61K 31/4545, A61K 9/14

(54) **A BIOAVAILABLE DOSAGE FORM OF LORATADINE**
BIOVERFÜGBARE DARREICHUNGSFORM VON LORATADIN
FORME GALENIQUE BIODISPONIBLE DE L'ORATADINE

(30) Priority: 17.07.2000 IN DE065100
(43) Date of publication of application: 23.04.2003
(73) Proprietor: RANBAXY LABORATORIES, LTD., New Delhi 110 019 (IN)
(72) Inventor: KUMAR, Pananchukunath Manoj, New Delhi 110 001 (IN); GUPTA, Dinsheet, Gurgaon 122 001 Haryana (IN); MALIK, Rajiv, New Delhi 110 019 (IN)
(74) Representative: Cronin, Brian Harold John
(86) International application number: PCT/IB2001/001098
(87) International publication number: WO 2002/005816

(56) References cited:
- WO-A-01/10415
- WO-A-99/62516

## Description

### FIELD OF THE INVENTION

The present invention relates to a bioavailable oral dosage form of loratadine.

### BACKGROUND OF THE INVENTION

Loratadine or ethyl 4-(8-chloro-5,6-dihydro-11H-benzo[5,6] cyclohepta [1,2-b] pyridin -11-ylidene)-1-piperidine carboxylate is useful as an antihistamine and is disclosed in U.S. Patent No. 4,282,233.

Loratadine is particularly advantageous for use of an antihistamine compared to other drugs of the same class as it is administered only once daily and has little or no sedative effects. It is therefore preferred for use by patients who have to perform mental or physical tasks requiring a high level of concentration. Loratadine however poses problems to the formulator as it has low solubility in water and therefore shows poor bioavailability characteristics.

### SUMMARY OF THE INVENTION

It is an objective of the present invention to provide a bioavailable oral dosage form of loratadine, that is bioequivalent to the commercially available formulation and falls within the prescribed limits set by various International Regulatory Agencies.

Accordingly, the present invention provides a bioavailable oral dosage form of loratadine, comprising reduced particle size loratadine, such that the average particle size ranges from about 0.1 microns to 15 microns and the average surface area falls between 1 and 2 m²/g.

It is observed that the particle size and the surface area of loratadine is critical in achieving bioequivalence against the commercially available formulation Claritin®, marketed by Schering Corporation. The particle size of the drug is reduced thereby increasing its surface area using any of the conventional milling techniques known in the art. These include the use of ball mill, cad mill, multi mill, air jet mill etc.

In preferred embodiments of the invention, the size of the drug is reduced such that the average particle size ranges between 1 microns to 10 microns. The surface area of the milled drug is maintained between 1 and 2 m²/g.

The milled drug is then formulated into a suitable dosage form such as tablet, capsule, syrup, suspension etc. In preferred embodiments the pharmaceutical dosage form is a tablet. The milled drug is mixed with pharmaceutically acceptable excipients such as fillers, binders and lubricants and further processed using processes conventionally known in the art such as direct compression, compaction or wet granulation.

The fillers employed in the present invention preferably comprise a pharmaceutically acceptable saccharides, including monosaccharides, a disaccharides, and polysaccharides, polyhydric alcohols, or cellulose ethers and mixtures thereof. Examples of suitable pharmaceutical fillers include sucrose, dextrose, lactose, microcrystalline cellulose, fructose, xylitol, sorbitol, hydroxypropyl methylcellulose, mixtures thereof and the like. However, it is preferred that a soluble pharmaceutical filler such as lactose, dextrose, sucrose, or mixtures thereof be used.

The binders used in accordance with the present invention are those conventionally used in the art and may be selected from: gums such as karaya gum and locus bean gum, starch, polyvinylpyrrolidones etc. The lubricants are selected from amongst those conventionally used in the art such as magnesium stearate, zinc stearate, talc, tristearin, tripalmitin, polyethylene glycols, waxes, hydrogenated oils, aerosil and mixtures thereof.

Investigations were conducted in order to determine the effect of particle size and surface area on the bioavailability of loratadine. The blood levels of the drug were compared with that of the commercially available formulation of loratadine sold under the trade name of Claritin®. The area under the plasma concentration (loratadine) vs time curve (AUC) was determined between time "0" and time "t" to give the AUC₍₀₋ₜ₎ values and was then extrapolated to infinity (α) to calculate the value till there was no more drug in the plasma. This value is reported as AUC_{(0-α)}. The maximum plasma concentration (Cmax) was also determined for each subject after each treatment.

### DETAILED DESCRIPTION OF THE INVENTION

### EXAMPLE 1

**Table 1.1**

| **Ingredients** | **Mg/Tab** |
|---|---|
| Loratadine | 10.0 |
| Lactose | 86.25 |
| Starch | 1.50 |
| Pregelatinised starch | 1.50 |
| Magnesium stearate | 0.75 |
| Total | 100.0mg |

The particle size of loratadine was 90% below 47.2 microns and 50% below 10.7 microns. The surface area was 1.126 m²/g. The active and the inactive excipients are mixed and compressed to tablets. The tablets released more than 90% of the content in 0.1 N HCl in USP apparatus I at 50 rpm.

The formulation was subjected to a two way cross over bioequivalence study with Claritin® (which was the reference product). Eighteen normal, male subjects were enrolled in each study. Whole blood samples were drawn at selected times following each treatment. Blood levels of the drug for both test and reference were determined and compared for the two critical parameters of AUC and Cmax (Table 1.1). Test is the formulation made according to present invention and reference is the formulation of loratadine sold under the trade name of Claritin®.

**Table 1.2**

| | AUC ₍₀₋ₜ₎ | AUC _{(0-α)} | Cₘₐₓ (µg/ml) |
|---|---|---|---|
| Test/ Reference (%) | 81.5 | 85 | 87.7 |

As can be seen when the particle size of loratadine was 90% below 47 microns, the formulation was only around 80% bioavailable as compared to the commercially available formulation of loratadine sold under the trade name Claritin®.

### EXAMPLE 2

The process of granulation was changed from direct compression to wet granulation to study its effect, if any, on the bioavailability of the drug.

**Table 2.1**

| **Ingredients** | **Mg/Tab** |
|---|---|
| Loratadine | 10.0 |
| Lactose | 86.50 |
| Starch | 1.50 |
| Pregelatinised starch | 1.50 |
| Magnesium stearate | 0.50 |
| Total | 100.0mg |

The particle size of loratadine was 90% below 47.2 microns and 50% below 10.7 microns and the surface area was 1.126 m²/g. The drug was mixed with the inactive excipients, and granulated using water. The granules were dried and the tablets were compressed. The tablets released 90% of the drug in 0.1 NHCl in USP apparatus 2 within 30 minutes.

This formulation was subjected to a two way cross over bioequivalence study with Claritin® on 18 normal male subjects as described in Example 1.

**Table 2.2**

| | AUC ₍₀₋ₜ₎ | AUC _{(0-α)} | Cₘₐₓ (µg/ml) |
|---|---|---|---|
| Test/ Reference (%) | 74.8 | 85.1 | 67.5 |

Once again the bioavailability of the drug in our formulation was low compared to that of Claritin®, indicating that changing the processing conditions does not improve the bioavailability characteristics of the drug.

### EXAMPLE 3

As loratadine of the larger particle size showed lower bioavailability as compared to the commercially available product Claritin®, it was decided to investigate the effect of reduction of particle size of the loratadine on its bioavailability.

**Table 3.1**

| **Ingredients** | **Mg/Tab** |
|---|---|
| Loratadine | 10.0 |
| Lactose | 79.75 |
| Starch | 7.5 |
| Pregelatinised starch | 2.0 |
| Magnesium stearate | 0.75 |
| Total | 100.0mg |

The particle size of loratadine was 90% below 10 microns and 50% below 5 microns and the surface area was 1.54 m²/g. The drug was mixed with the inactive excipients, granulated using water, the granules were dried, lubricated and then compressed to tablets.

The tablets released more than 90% of the drug in 0.1 NHCl in USP apparatus 2 within 30 minutes. The formulation was subjected to a two way crossover bioequivalence study on 20 healthy, male subjects as described in Example 1.

**Table 3.2**

| | AUC ₍₀₋ₜ₎ | AUC _{(0-α)} | Cₘₐₓ (µg/ml) |
|---|---|---|---|
| Test/ Reference (%) | 100.7 | 91.7 | 95.5 |

As can be seen from the data, reduction in particle size of the drug led to a dramatic increase in the bioavailability of loratadine to equal that of the commercially available product Claritin®.

The next example further illustrates the importance of particle size and increased surface area on the bioavailability of loratadine.

### EXAMPLE 4

**Table 4.1**

| **Ingredients** | **Mg/Tab** |
|---|---|
| Loratadine | 10.0 |
| Lactose | 80.60 |
| Starch | 7.5 |
| Pregelatinised starch | 2.0 |
| Magnesium stearate | 0.50 |
| Total | 100.0mg |

The particle size of loratadine was almost similar to that used in example 3 i.e. 90% below 9 microns and 50% below 6 microns but the surface area at 2.042 m²/g was larger than that of loratadine used in Example 3. All the active and inactives were mixed and granulated using water. The granules were dried and compressed to tablets. The tablets released 90% of the drug in 0.1 NHCl in USP apparatus 2 within 45 minutes.

The formulation was subjected to a two way crossover study on 11 normal, healthy, male subjects as described in Example 1.

**Table 4.2**

| | AUC ₍₀₋ₜ₎ | AUC _{(0-α)} | Cₘₐₓ (µg/ml) |
|---|---|---|---|
| Test/Reference (%) | 134 | 124 | 130 |

Increase in the surface area together with the reduction of particle size caused a dramatic increase in the bioavailability of the drug to almost 30% greater than that of the commercially available reference product.

These results emphasize the criticality of particle size and surface area of loratadine on its bioavailability.

## Claims

1. A bioavailable oral dosage form of loratadine comprising loratadine having an average particle size ranging from about 0.1 microns to about 15 microns and having a surface area ranging from between 1 and 2.5 m²/g.

2. The bioavailable oral dosage form of claim 1, wherein the particle size of loratadine is between about 1 micron to about 10 microns.

3. The bioavailable oral dosage form of claim 1, wherein the surface area of loratadine is between 1.25 and 2.0 m²/g.

4. The bioavailable oral dosage form of claim 1, wherein the drug is mixed with other pharmaceutically acceptable fillers, binders, and lubricants.

5. The bioavailable oral dosage form of claim 4, wherein the fillers used are selected from: saccharides, polyhydric alcohols, celluloses, and cellulose ethers.

6. The bioavailable oral dosage form of claim 4, wherein the fillers are selected from: lactose, dextrose, sucrose, microcrystalline celluloses, hydroxypropyl methyl cellulose, and mixtures thereof.

7. The bioavailable oral dosage form of claim 4, wherein the binders are selected from: starch, polyvinylpyrrolidone, and gums.

8. The bioavailable oral dosage form of claim 4, wherein the lubricants are selected from: talc, magnesium strearate, zinc stearate, tristearin, tripalmitin, polyethylene glycol, waxes, aerosil, and mixtures thereof.

9. The bioavailable oral dosage form of claim 1, wherein the dosage form is formulated as a tablet, capsule or suspension.

10. A process for the preparation of a bioavailable oral dosage form of loratadine comprising the step of milling said loratadine to reduce the particle size such that the average particle size ranges from about 0.1 microns to about 15 microns and the surface area ranges from between 1 and 2.5 m²/g.

11. The process as described in claim 10, wherein the particle size of loratadine is between about 1 micron to about 10 microns.

12. The process as described in claim 10, wherein the surface area of loratadine is between 1.25 and 2.0 m²/g.

13. The process as described in claim 10, wherein the milled drug is mixed with other pharmaceutically acceptable fillers, binders, and lubricants.

14. The process as described in claim 13, wherein the fillers used are selected from: saccharides, polyhydric alcohols, celluloses, and cellulose ethers.

15. The process as described in claim 13, wherein the fillers are selected from: lactose, dextrose, sucrose, microcrystalline celluloses, hydroxypropyl methyl cellulose, and mixtures thereof.

16. The process as described in claim 13, wherein the binders are selected from: starch, polyvinylpyrrolidone, and gums.

17. The process as described in claim 13, wherein the lubricants are selected from: talc, magnesium strearate, zinc stearate, tristearin, tripalmitin, polyethylene glycol, waxes, aerosil, and mixtures thereof.

18. The process as described in claim 10, wherein the dosage form is formulated as a tablet, capsule or suspension.

## Patentansprüche

1. Bioverfügbare orale Arzneiform von Loratadin umfassend Loratadin mit einer durchschnittlichen Teilchengröße im Bereich von etwa 0,1 Mikron bis etwa 15 Mikrons und mit einer Oberfläche im Bereich von 1 bis 2,5 m²/g.

2. Bioverfügbare orale Darreichungsform nach Anspruch 1, wobei die Teilchengröße von Loratadin zwischen etwa 1 Mikron und etwa 10 Mikrons liegt.

3. Bioverfügbare orale Darreichungsform nach Anspruch 1, wobei die Oberfläche von Loratadin zwischen 1,25 und 2,0 m²/g liegt.

4. Bioverfügbare orale Darreichungsform nach Anspruch 1, wobei der Wirkstoff mit anderen pharmazeutisch akzeptablen Füllstoffen, Bindemitteln und Gleitmitteln vermischt ist.

5. Bioverfügbare orale Darreichungsform nach Anspruch 4, wobei die verwendeten Füllstoffe ausgewählt sind aus: Sacchariden, polyhydrische Alkohole, Zellulosen und Zelluloseethern.

6. Bioverfügbare orale Darreichungsform nach Anspruch 4, wobei die Füllstoffe ausgewählt sind aus: Laktose, Dextrose, Sucrose, mikrokristalline Zellulosen, Hydroxypropylmethylzellulose, sowie Mischungen davon.

7. Bioverfügbare orale Darreichungsform nach Anspruch 4, wobei die Bindemittel ausgewählt sind aus: Stärke, Polyvinylpyrrolidon und Gummen.

8. Bioverfügbare orale Darreichungsform nach Anspruch 4, wobei die Gleitmittel ausgewählt sind aus: Talkum, Magnesiumstearat, Zinkstearat, Tristearin, Tripalmitin, Polyethylenglykol, Wachse, Aerosil und Mischungen davon.

9. Bioverfügbare orale Darreichungsform nach Anspruch 1, wobei die Darreichungsform als Tablette, Kapsel oder Suspension formuliert ist.

10. Verfahren zur Herstellung einer bioverfügbaren oralen Darreichungsform von Loratadin, umfassend den Schritt des Mahlens von Loratadin um die Teilchengröße so zu verringern, dass die durchschnittliche Teilchengröße im Bereich von etwa 0,1 Mikron bis etwa 15 Mikrons und die Oberfläche im Bereich zwischen 1 und 2,5 m²/g liegt.

11. Verfahren nach Anspruch 10, wobei die Teilchengröße von Loratadin zwischen etwa 1 Mikron und etwa 10 Mikrons liegt.

12. Verfahren nach Anspruch 10, wobei die Oberfläche von Loratadin zwischen 1,2 und 2,0 m²/g liegt.

13. Verfahren nach Anspruch 10, wobei der gemahlene Wirkstoff mit anderen pharmazeutisch akzeptablen Füllstoffen, Bindemitteln und Gleitmitteln vermischt wird.

14. Verfahren nach Anspruch 13, wobei die verwendeten Füllstoffe ausgewählt sind aus: Sacchariden, polyhydrischen Alkoholen, Zellulosen und Zelluloseethern.

15. Verfahren nach Anspruch 13, wobei die Füllstoffe ausgewählt sind aus: Laktose, Dextrose, Sucrose, mikrokristalline Zellulosen, Hydroxypropylmethylzellulose sowie Mischungen davon.

16. Verfahren nach Anspruch 13, wobei die Bindemittel ausgewählt sind aus: Stärke, Polyvinylpyrrolidon und Gummen.

17. Verfahren nach Anspruch 13, wobei die Gleitmittel ausgewählt sind aus: Talkum, Magnesiumstearat, Zinkstearat, Tristearin, Tripalmitin, Polyethylenglykol, Wachse, Aerosil und Mischungen davon.

18. Verfahren nach Anspruch 10, wobei die Darreichungsform als Tablette, Kapsel oder Suspension formuliert ist.

## Revendications

1. Forme galénique orale biodisponible de loratadine comprenant de la loratadine ayant une granulométrie moyenne s'étendant d'environ 0,1 micromètre à environ 15 micromètres et ayant une surface s'étendant entre 1 et 2,5 m²/g.

2. Forme galénique orale biodisponible de la revendication 1, dans laquelle la granulométrie de la loratadine est entre environ 1 micromètre et environ 10 micromètres.

3. Forme galénique orale biodisponible de la revendication 1, dans laquelle la surface de la loratadine est entre 1,25 et 2,0 m²/g.

4. Forme galénique orale biodisponible de la revendication 1, dans laquelle le médicament est mélangé avec d'autres charges, liants et lubrifiants pharmaceutiquement acceptables.

5. Forme galénique orale biodisponible de la revendication 4, dans laquelle les charges utilisées sont choisies à partir de saccharides, polyalcools, celluloses et éthers de cellulose.

6. Forme galénique orale biodisponible de la revendication 4, dans laquelle les charges sont choisies à partir de lactose, dextrose, sucrose, celluloses microcristallines, hydroxypropylméthylcellulose et mélanges de ceux-ci.

7. Forme galénique orale biodisponible de la revendication 4, dans laquelle les liants sont choisis à partir d'amidon, polyvinylpyrrolidone et gommes.

8. Forme galénique orale biodisponible de la revendication 4, dans laquelle les lubrifiants sont choisis à partir de talc, stéarate de magnésium, stéarate de zinc, tristéarine, tripalmitine, polyéthylèneglycol, cires, aérosil et mélanges de ceux-ci.

9. Forme galénique orale biodisponible de la revendication 1, dans laquelle la forme galénique est formulée en tant que comprimé, gélule ou suspension.

10. Procédé pour la préparation d'une forme galénique orale biodisponible de loratadine comprenant l'étape de broyer ladite loratadine pour réduire la granulométrie de telle sorte que la granulométrie moyenne varie d'environ 0,1 micromètre à environ 15 micromètres et que la surface varie de 1 à 2,5 m²/g.

11. Procédé tel que décrit dans la revendication 10, dans lequel la granulométrie de la loratadine est entre environ 1 micromètre et environ 10 micromètres.

12. Procédé tel que décrit dans la revendication 10, dans lequel la surface de la loratadine est entre 1,25 et 2,0 m²/g.

13. Procédé tel que décrit dans la revendication 10, dans lequel le médicament broyé est mélangé avec d'autres charges, liants et lubrifiants pharmaceutiquement acceptables.

14. Procédé tel que décrit dans la revendication 13, dans lequel les charges utilisées sont choisies à partir de saccharides, polyalcools, celluloses et éthers de cellulose.

15. Procédé tel que décrit dans la revendication 13, dans lequel les charges sont choisies à partir de lactose, dextrose, sucrose, celluloses microcristallines, hydroxypropylméthylcellulose et mélanges de ceux-ci.

16. Procédé tel que décrit dans la revendication 13, dans lequel les liants sont choisis à partir d'amidon, polyvinylpyrrolidone et gommes.

17. Procédé tel que décrit dans la revendication 13, dans lequel les lubrifiants sont choisis à partir de talc, stéarate de magnésium, stéarate de zinc, tristéarine, tripalmitine, polyéthylèneglycol, cires, aérosil et mélanges de ceux-ci.

18. Procédé tel que décrit dans la revendication 10, dans lequel la forme galénique est formulée en tant que comprimé, gélule ou suspension.
